(19) 

(11) **EP 4 537 863 B1**

(12) **EUROPEAN PATENT SPECIFICATION**

(45) Date of publication and mention of the grant of the patent:
**23.09.2026 Bulletin 2026/39**

(21) Application number: **23203654.1**

(22) Date of filing: **13.10.2023**

(51) International Patent Classification (IPC):
***A61M 1/16*** *(2006.01)* ***A61M 1/34*** *(2006.01)*
***A61M 1/36*** *(2006.01)*

(52) Cooperative Patent Classification (CPC):
**A61M 1/3496; A61M 1/1607; A61M 1/1609; A61M 1/1619; A61M 1/165; A61M 1/3424; A61M 1/3609; A61M 1/3672;** A61M 2205/3306; A61M 2205/70; A61M 2230/20

(54) **EXTRACORPOREAL BLOOD TREATMENT APPARATUS**

EXTRAKORPORALE BLUTBEHANDLUNGSVORRICHTUNG

APPAREIL DE TRAITEMENT EXTRACORPOREL DU SANG

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**16.04.2025 Bulletin 2025/16**

(73) Proprietor: **Gambro Lundia AB**
**226 43 Lund (SE)**

(72) Inventor: **POUCHOULIN, Dominique**
**01390 Tramoyes (FR)**

(74) Representative: **PGA S.p.A., Milano, Succursale di Lugano**
**Via Castagnola, 21c**
**6900 Lugano (CH)**

(56) References cited:
**EP-A2- 1 029 554**
**FR-A1- 2 712 822**
**US-A- 5 685 988**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 537 863 B1

**Description**

BACKGROUND

The present disclosure relates to an extracorporeal blood treatment apparatus configured to perform extracorporeal blood treatments, such as dialysis fluid treatments.

Due to various causes, a person's renal system can fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with dialysis. Dialysis removes waste, toxins and excess water from the body that normal functioning kidneys would otherwise remove. Dialysis treatment for replacement of kidney functions is critical to many people because the treatment is lifesaving. Kidney failure therapies include for instance Hemodialysis (HD), Hemofiltration (HF) Hemodiafiltration (HDF).

The apparatus of the present invention may be used in the context of continuous renal replacement therapies (CRRT) with or without anticoagulation, for example CRRT with or without systemic anticoagulation (e.g., heparin) / with or without regional anticoagulation (e.g., citrate).

Irrespective of the type of therapy and/or treatment referred to, in-line sensing technology plays an important role in the efficient functioning of the extracorporeal blood treatment apparatuses.

For instance, the general concept of providing a single (e.g., concentration) sensor placed on a by-pass line running between fresh dialysate and effluent and configured for measuring a property of the fresh dialysis fluid and the spent dialysis fluid is known from some prior art documents.

US2014190876 refers to systems and methods for utilizing a sorbent cartridge and quantifying chemistry changes that occur as dialysate passes through material layers of the cartridge as a means to determine solute concentrations in the solution flowing through the cartridge. The systems and methods disclosed can be used for managing blood urea and quantifying urea clearance during dialysis therapy also including hemodialysis, hemodiafiltration, hemofiltration and peritoneal dialysis. A fluid circuit equipped with a single conductivity sensor is active on a by-pass circuit parallel to the dialyzer. The by-pass circuit allows the diversion of a portion of the dialysate around the dialyzer to rejoin the flow path in the post-dialyzer flow path.

US5399157 discloses a method and a device for checking the operation of one sensor situated in a dialysis liquid circuit of an artificial kidney. The method includes causing a reference liquid of known characteristics to circulate in the extracorporeal blood circuit, taking a measurement to check at least one characteristic of the dialysis liquid through the sensor, calculating the theoretical value of at least one characteristic of the reference liquid on the basis of the measurement, comparing the calculated theoretical value with the known value of the said characteristic of the reference liquid, concluding that the sensor is working properly after the measurement has been taken when the calculated theoretical value is substantially equal to the known value.

US5744031 discloses an artificial kidney including a measurement structure for measuring a physical characteristic of a fresh dialysis liquid and of used liquid. The measurement structure is disposed in a line portion common to a branch circuit to the feeder line of fresh dialysis liquid and to a branch circuit to the discharge line for the used liquid. An occluding structure permits the liquid to circulate exclusively in one or the other branch circuit. Due to this arrangement, it is possible to obtain the value of the physical characteristics of a patient's blood by calculation and to adjust the operation of the kidney permanently to a therapeutic objective set by the physician.

US6623442 discloses a dialysis apparatus including a dialysis liquid circuit for circulating sodium chloride and sodium bicarbonate through a haemodialyzer and a circuit for infusing a patient with a solution containing at least one ionic substance. A dialysance detector determines the actual dialysance of the haemodialyzer for sodium and a flow rate detector determines the flow rate of infusion solution such that the concentration of the substance inside the patient's body tends towards a desired concentration as a function of the dialysance, the concentration of the substance in the infusion solution and the desired concentration.

US2014098359 discloses a method for monitoring a blood treatment of a patient comprising the steps of irradiating a sample of a dialysis liquid used in the treatment with irradiation light of at least a first irradiation wavelength, detecting light emitted by the irradiated sample in at least a first detection wavelength, the detection wavelength being different from the first irradiation wavelength, and determining the presence and/or concentration of an analyte in the sample on the basis of the detected light.

WO2021205389 discloses a method and a system for providing a personalized hemodialysis treatment to a subject. The method includes obtaining concentration of electrolytes and of metabolic content in blood sample flowing into and out of dialyzer through first blood bypass tube and second blood bypass tube in which a first sensor and second sensor are arranged. The method includes obtaining concentration of electrolytes and metabolic content in dialysate fluid flowing into and out of dialyzer through a first dialysate duct and a second dialysate duct. The first dialysate duct and second dialysate duct are arranged to pass through a third sensor and a fourth sensor. Variations are identified in concentration

obtained for electrolytes and metabolic content in blood sample with respect to concentration obtained for electrolytes and metabolic content in dialysate fluid, respectively.

Documents US5685988A1 and EP1029554A2 disclose extracorporeal blood treatment apparatuses comprising a by-pass line.

It is an aim achieved by the present disclosure to provide an extracorporeal blood treatment apparatus configured for obtaining concentration measures of substances in fluids flowing in or out of the filtration unit (like the supply fluid, the effluent fluid, etc.) with a simple and reliable structure.

**It** is also an aim achieved by the present disclosure to provide an extracorporeal blood treatment apparatus configured for obtaining concentration measures on the fluid lines with a straightforward design resulting less complex and less expensive than the traditional prior art extracorporeal blood treatment apparatuses.

**It** is also an aim achieved by the present disclosure to provide an extracorporeal blood treatment apparatus configured for providing accurate measures of the solute removal rate and of clearance or dialysance of a substance with a simple and reliable structure. Indeed, the solute removal rate J is measured with a better accuracy from the supply/effluent side than on the blood side. This better accuracy is driven by a larger concentration difference between Ceff and Csup in comparison to the blood inlet-outlet concentration difference and a more accurate estimate/measurement of the fluid flow rate (typically from scales on the fluid side versus volumetric flow from the peristaltic blood pump). Another positive aspect of measuring J via supply/effluent data is that it does not require for any assumptions about the solute distribution and possible mass transfer between red blood cells (RBCs) and plasma.

It is another aim achieved by the present disclosure to provide an extracorporeal blood treatment apparatus easier to be monitored than the traditional prior art extracorporeal blood treatment apparatuses.

It is yet a further aim achieved by the present disclosure to provide an extracorporeal blood treatment apparatus through which one or more solute concentration in the fluids is monitored a plurality of times during a treatment allowing a better treatment accuracy than the one achieved by the traditional prior art extracorporeal blood treatment apparatuses.

Additional features and advantages are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not have to have all of the advantages listed herein and individual advantageous embodiments are expressly contemplated separately. Moreover, it should be noted that the language used in the specification has been selected principally for readability and instructional purposes, and not to limit the scope of the disclosed subject matter.

An extracorporeal blood treatment apparatus according to the present invention comprises the technical features as defined in independent claim 1.

## BRIEF DESCRIPTION OF THE FIGURES

Figure 1 is a schematic view of an extracorporeal blood treatment apparatus according to a first embodiment of the present invention;

Figure 2 is a schematic view of a fluid circuit of an extracorporeal blood treatment apparatus according to a second embodiment of the present invention;

Figure 3 is a schematic view of a fluid circuit of an extracorporeal blood treatment apparatus according to a third embodiment of the present invention;

Figure 4 is a schematic view of a fluid circuit of an extracorporeal blood treatment apparatus according to a fourth embodiment of the present invention;

Figure 5 is a first block diagram representing a monitoring method for monitoring at least one substance into a fluid according to the present application;

Figure 6 is second first block diagram representing another monitoring method for monitoring at least one substance into a fluid according to the present application;

Figure 7 is third first block diagram representing a further monitoring method for monitoring at least one substance into a fluid according to the present application;

Figure 8 is a variant embodiment of a part of the fluid circuit of the present invention;

Figures 9 and 10 show further variants of the fluid circuit of the invention.

## DETAILED DESCRIPTION

Extracorporeal blood treatment (for example, but not exclusively, renal failure dialysis) may be used in patients with rapidly developing loss of kidney function, called acute renal failure or slowly worsening kidney function, called Stage

5 chronic kidney disease (or end-stage renal disease). In the following description, some embodiments of extracorporeal blood treatment apparatuses will be firstly described being suitable, or designed, principally (though not exclusively) for intensive care treatments.

With reference to the attached figures, the numeral 1 globally refers to an extracorporeal blood treatment apparatus which may be used for performing Hemodialysis ("HD"), Hemofiltration ("HF"), Hemodiafiltration ("HDF") as well as the treatments performed during continuous renal replacement therapies (CRRT).

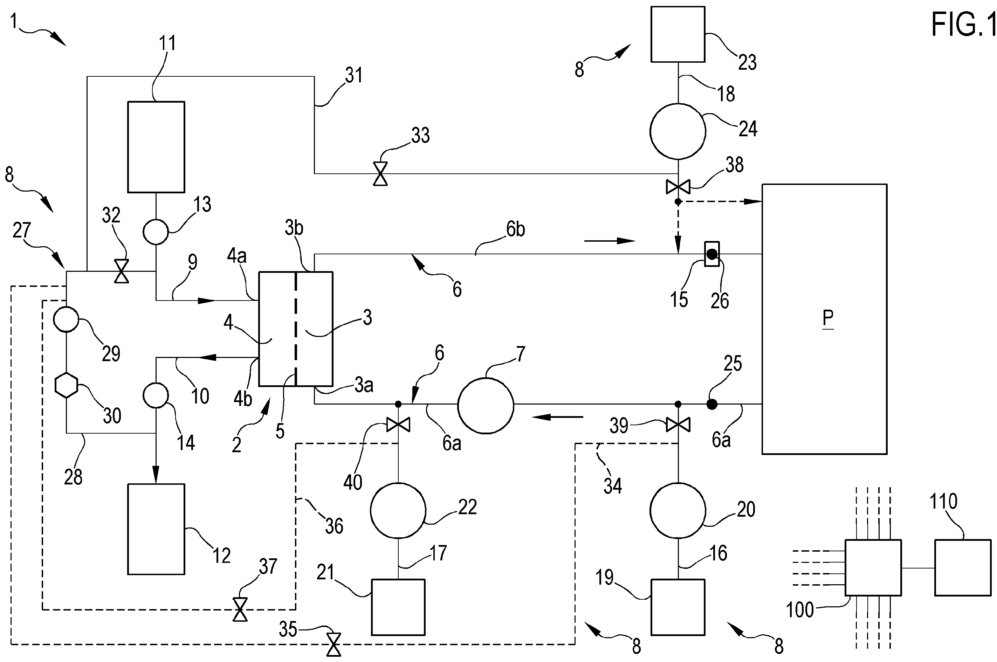

With reference to figure 1, the extracorporeal blood treatment apparatus 1 comprises a filtration unit 2, of the type having a primary chamber 3 and a secondary chamber 4 separated by a semipermeable membrane 5, an extracorporeal blood circuit 6 comprising a blood withdrawal line 6a connected to an inlet 3a of the primary chamber 3 of the filtration unit 2, and a blood return line 6b connected to an outlet 3b of the primary chamber 3 of the filtration unit 2. The extracorporeal blood circuit 6 is configured to be connected to a patient "P". A blood pump 7 is active on the extracorporeal blood circuit 6 to pump blood through said extracorporeal blood circuit 6, from the patient "P" across the blood withdrawal line 6a to the filtration unit 2 and from the filtration unit 2 across the blood return line 6b back to the patient "P".

The extracorporeal blood treatment apparatus 1 further comprises a fluid circuit 8 comprising a dialysis line 9 and an effluent line 10 connected to the secondary chamber 4. The dialysis line 9 has one end connected to an inlet 4a of the secondary chamber 4 and one end connected to a source of dialysis fluid 11, e.g. a bag or a preparation device of fresh dialysis fluid. The effluent line 10 has one end connected to an outlet 4b of the secondary chamber 4 and one end connected to a drainage 12. A dialysis pump 13 is active on the dialysis line 9 and is able to pump fluid to the second chamber 4. An effluent pump 14 is active on the effluent line 10 and is able to draw fluid from the second chamber 4.

An air detector, not shown, and an air separator, such as a deaeration chamber 15, may be present on the blood return line 6b.

The fluid circuit 8 may also comprise a replacement infusion circuit comprising at least one replacement infusion line connected to the extracorporeal blood circuit 6 or directly to the patient "P", a replacement infusion pump active on the replacement infusion line, a source of replacement solution connected to one end of the replacement infusion line. The replacement infusion circuit shown in the embodiment of Figure 1 comprises a pre-blood pump line 16, a pre-infusion line 17 and a post-infusion line 18.

The dialysis line 9, pre-blood pump line 16, pre-infusion line 17 and post-infusion line 18 are supply lines of the fluid circuit 8.

The pre-blood pump line 16 is connected to the blood withdrawal line 6a upstream of the blood pump 7 and to a first source 19 of replacement solution, e.g. a bag. A pre-blood pump 20 is located on the pre-blood pump line 16 and is able to pump fluid from the first source 19 to the blood circuit.

The pre-infusion line 17 is connected to the blood withdrawal line 6a downstream of the blood pump 7 and upstream of the filtration unit 2 and to a second source 21 of replacement solution, e.g. a bag. A pre-infusion pump 22 is located on the pre-infusion line 17 and is able to pump fluid from the second source 21 to the blood circuit.

The post-infusion line 18 is connected to the blood return line 6b downstream of the filtration unit 2 and to a third source 23 of replacement solution, e.g. a bag. A post-infusion pump 24 is located on the post-infusion line 18 and is able to pump fluid from the third source 23 to the blood circuit.

The fluid circuit 8 may also comprise one or more further fluid line/s, not shown, connected to the blood circuit and to a source of at least one compensation substance or of an anticoagulant, and a pump or syringe configured to deliver a flow rate of the compensation substance, such as potassium or bicarbonate, etc..

A withdrawal pressure sensor 25 is configured to detect a pressure at a measurement location in the blood withdrawal line 6a. A return pressure sensor 26 is configured to detect a pressure at a measurement location in the blood return line 6b. The withdrawal pressure sensor 25 and the return pressure sensor 26 may comprise pressure pods in the blood withdrawal line 6a and blood return line 6b. The return pressure sensor 26 may be operatively coupled to the deaeration chamber 15, as shown in Figure 1. A monitor valve, not shown, may be present on the blood return line 6b, downstream the deaeration chamber 15.

A control unit 100 is configured to control the extracorporeal blood treatment apparatus 1 to perform an extracorporeal blood treatment on the patient "P".

The control unit 100 is connected and drives the blood pump 7, the dialysis pump 13, the effluent pump 14, the pre-blood pump 20, the pre-infusion pump 22 and the post-infusion pump 24 to regulate a blood flow rate "Qb" in the blood circuit, a supply flow rate crossing the dialysis line 9, an effluent flow rate crossing the effluent line 10, an infusion flow rate crossing the pre-blood pump line 16, an infusion flow rate crossing the pre-infusion line 17, an infusion flow rate crossing the post-infusion line 18. Through the control of the supply flow rate crossing the dialysis line 9 and/or of the effluent flow rate crossing the effluent line 10, the control unit 100 is also configured to control/regulate a filtration flow rate in the filtration unit 2 and/or a patient fluid removal rate. The control unit 100 is also connected to the withdrawal pressure sensor 25 and to the return pressure sensor 26 to receive signals correlated to pressure values from these sensors 25, 26.

The control unit 100 may be an electronic control unit comprising at least a CPU, a memory and input/output devices. The control unit 100 comprises or is connected to an interface 110 configured to display data and/or allow a user to

input data. For instance, the interface comprises a display, e.g. a touch screen, and/or buttons or a keyboard.

The apparatus 1 further comprises a sampling circuit 27 comprising a by-pass line 28 having one end connected to the dialysis line 9 of the fluid circuit 8 and another end connected to the effluent line 10 of the fluid circuit 8. A fluid pump assembly 29 is active on the sampling circuit 27. A concentrate sensor 30 is placed on the by-pass line 28 and is configured for monitoring the concentration of at least one substance detectable into one of the fluids crossing the by-pass line 28, i.e. one or more fluids crossing the fluid circuit 8. In the embodiment of Figure 1, the fluid pump assembly 29 is a single reversible and occlusive pump acting on the by-pass line 28 and the concentrate sensor 30 is positioned between the fluid pump assembly 29 and the dialysis line 9. In other embodiments, not shown in drawings, the fluid pump assembly 29 may be positioned between the concentrate sensor 30 and the dialysis line 9. The sampling circuit 27 together with the fluid circuit 8 and the extracorporeal blood circuit 6 may be part of a disposable set which may be mounted to and dismounted from a machine for extracorporeal blood treatment comprising actuating devices, like the pumps, and electronic devices, e.g. the control unit 100. In this regard, the sampling circuit 27 may be a circuit already integrated into the disposable set (being monolithic with the fluid circuit 8) or separate from and connectable to the fluid circuit 8 and/or the extracorporeal blood circuit 6. The extracorporeal blood treatment apparatus 1 comprises the machine for extracorporeal blood treatment and the disposable set.

The concentrate sensor 30 may be an optical sensor which provides for continuous measurements and may be permanently in contact with the fluid to be tested (e.g. an optical sensor using fluorescence). Other type of sensors may alternatively be used such as ion selective sensors. In general, the concentration sensor 30 for measuring the concentration of a specific substance or several different concentrations for several substances of interest may be of different types.

For example, the concentration sensor 30 may be an ultraviolet (UV) absorbance sensor that measures the absorbance of UV light by the fluid. As waste products like urea and creatinine are removed from the blood, they increase the UV absorbance of the dialysate. By monitoring these changes, clinicians can gauge the efficacy of the dialysis treatment.

Alternatively, the concentration sensor 30 may be an ion selective electrode (ISE) to measure specific ions in a solution. They are used to monitor specific electrolytes in the dialysate or the patient's blood, such as potassium or calcium.

It may be a biosensor, namely a device that uses a biological component (like an enzyme) to detect specific substances. It has potential for measuring specific molecules of interest.

As mentioned the concentration sensor 30 may be an optical sensors that employs light properties to measure concentrations of specific solutes. For instance, some optical sensors can detect changes in color or fluorescence related to specific solute concentrations.

It may be a redox potential sensor to measure the reduction-oxidation potential of the dialysate. It may give insight into the chemical properties and reactions occurring in the dialysate.

The control unit 100 is connected and controls the fluid pump assembly 29 and is connected to the concentrate sensor 30 to execute a monitoring method for monitoring the concentration of at least one substance in one of the fluids crossing the fluid circuit 8. Since concentration of a substance is desired, the concentration sensor is capable of providing the specific concentration of such a substance (e.g., calcium) without providing a value that includes or is influenced by other solutes, such as sodium.

To this aim, the control unit 100 is configured to drive the fluid pump assembly 29 either in a first activated condition or in a second activated condition. In the first activated condition, the fluid pump assembly 29 causes the fluid in the by-pass line 28 to flow in one direction, from the dialysis line 9 towards the effluent line 10. In the second activated condition, the fluid pump assembly 29 causes the fluid in the by-pass line 28 to flow in an opposite direction, from the effluent line 10 towards the dialysis line 9. The fluid pump assembly 29 may be also set in an inactive state in which said fluid pump assembly 29 is stopped and/or at least one valve isolates the sampling circuit 27 from the secondary chamber 4 and from the extracorporeal blood circuit 6.

The control unit 100 is also configured to receive signals from the concentrate sensor 30 and to determine and monitor the concentration of the at least one substance crossing the by-pass line 28.

The apparatus 1 of the embodiment of Figure 1 comprises also a feed line 31 having one end connected to the post-infusion line 18, at a location downstream of the post-infusion pump 24, and another end connected to the by-pass line 28, at a connection location between the dialysis line 9 and fluid pump assembly 29. A valve 32 is operatively active on the by-pass line 28 between the dialysis line 9 and the feed line 31. A feed valve 33 is operatively active on the feed line 31.

The apparatus 1 of the embodiment of Figure 1 may also comprise (dashed lines) another feed line 34 having one end connected to the pre-blood pump line 16, at a location downstream of the pre-blood pump 20, and another end connected to the by-pass line 28, at a connection location between the dialysis line 9 and the fluid pump assembly 29. Another feed valve 35 is operatively active on the other feed line 34.

The apparatus 1 of the embodiment of Figure 1 may also comprise (dashed lines) a further feed line 36 having one end connected to the pre-infusion line 17, at a location downstream of the pre-infusion pump 22, and another end connected to the by-pass line 28, at a connection location between the dialysis line 9 and the fluid pump assembly 29. A

further feed valve 37 is operatively active on the further feed line 36.

In an embodiment not shown, a pre- and post-infusion line may be provided, i.e., an infusion line having branches that direct the fluid to the blood withdrawal and to the blood return lines. A corresponding feed line having one end connected to the pre- and post-infusion line, at a location downstream of the pump 20 acting on said line (either on the common tract or on the pre-infusion branch or on the post infusion branch), and another end connected to the by-pass line 28.

A first valve 38, a second valve 39 and a third valve 40 may be operatively active respectively on the post-infusion line 18 (between the feed line 31 and the blood return line 6b), on the pre-blood pump line 16 (between the another feed line 34 and the blood withdrawal line 6a) and on the pre-infusion line 17 (between the further feed line 36 and the blood withdrawal line 6a).

The control unit 100 is configured to run software routines configured to determine the concentration of the at least one substance or to monitor the at least one substance during the extracorporeal blood treatment or by interrupting temporarily the extracorporeal blood treatment. The control unit 100 is configured to run software routines configured to perform several monitoring methods.

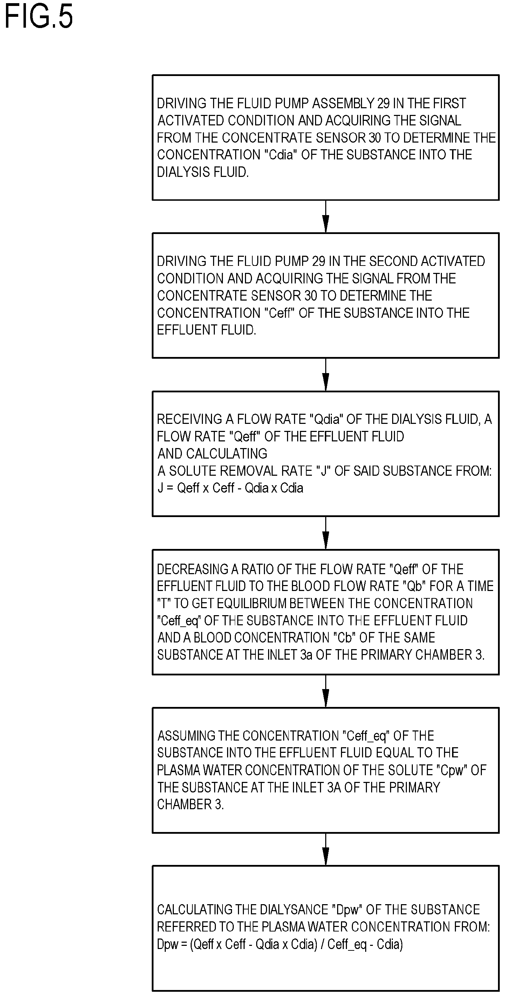

According to an example of monitoring method (Figure 5), in the course of a Hemodialysis (HD or HDF) extracorporeal blood treatment, the control unit 100 is configured to estimate a filter clearance or dialysance "K/D" of a substance by employing only the concentrate sensor 30 on the by-pass line 28. No other concentrate sensor on the extracorporeal blood circuit 6 is provided or employed for monitoring the concentration of said substance into blood and for calculating the clearance or dialysance "K/D".

Filter dialysance "K/D" is calculated in the general case where the solute concentration is non-zero in the dialysis fluid. In the scenario where the solute of interest is not present in the dialysis fluid (Cdia = 0), then dialysance "K/D" is called clearance. This is the typical case when dealing with urea, uric acid, creatinine, but may be not if looking to glucose (which may be present or not into the supply fluid).

The control unit 100 closes or keeps closed the feed valves 33, 35, 37, opens the valve 32 and drives the fluid pump assembly 29 in the first activated condition to make the dialysis fluid flow from the dialysis line 9 to the effluent line 10 through the by-pass line 28. The dialysis fluid interacts with the concentrate sensor 30 and the control unit 100 acquires the signal from the concentrate sensor 30 and determines the concentration "Cdia" of the substance into the dialysis fluid.

Then the control unit 100 drives the fluid pump assembly 29 in the second activated condition to make the effluent fluid flow from the effluent line 10 to the dialysis line 9 through the by-pass line 28 and acquires the signal from the concentrate sensor 30 to determine the concentration "Ceff" of the substance into the effluent fluid.

All fluid flow rates are usually well controlled and known to the system. Therefore, the control unit 100 receives a flow rate "Qdia" of the dialysis fluid (set or measured), a flow rate "Qeff" of the effluent fluid (set or measured) and calculates a solute removal rate "J" of said substance from the following equation:

$$1) \quad J = Qeff \times Ceff - Qdia \times Cdia$$

The control unit 100 decreases (with respect to prescriptions values for the extracorporeal blood treatment) a ratio of the flow rate "Qeff" of the effluent to the blood flow rate "Qb" for a time "t", in order to secure equilibrium between the concentration "Ceff_eq" of the substance into the effluent fluid and a blood concentration "Cb" of the same substance at the inlet 3a of the primary chamber 3. Since in HD mode Qeff is closely related to Qdia (Qeff=Qdia + Qpfr) a ratio of the flow rate "Qdia" of the dialysis fluid to the blood flow rate "Qb" may be decreased.

For instance, the blood flow rate "Qb" is 200 ml/min, the prescription value of the flow rate "Qdia" of the dalysate fluid is 2500 ml/h and is decreased to a low value of 1000 ml/h for a time "t" of 5 min. A ratio of the low value of the flow rate "Qdia" of the dialysate fluid to the blood flow rate "Qb" is decreased from 0.208 to 0.083.

Once the equilibrium is reached, it is assumed that the concentration "Ceff_eq" of the substance into the effluent fluid equals the plasma water concentration of the solute "Cpw" of the substance at the inlet 3a of the primary chamber 3 and the control unit 100 calculates the dialysance "Dpw" of the substance referred to the plasma water concentration through the following formula:

$$Dpw = J / (Cpw - Cdia) = J / (Ceff_eq - Cdia) = (Qeff \times Ceff - Qdia \times Cdia) / (Ceff_eq - Cdia) \qquad 3)$$

If the intent is to report the dialysance "Dp" referring to the plasma concentration, plasma solute concentration at the filter inlet can be estimated as Cp = Fp x Cpw, wherein "Fp" is the plasma water fraction, leading to:

$$Dp = J / (Cp - Cdia) = J / (Fp \times Ceff_eq - Cdia) = (Qeff \times Ceff - Qdia \times Cdia) / (Fp \times Ceff_eq - Cdia) \qquad 4)$$

Once the effluent concentration of the solute at equilibrium "Ceff_eq" has been calculated, the control unit 100

stops the fluid pump assembly 29 and may also close the valve 32 and then control the effluent pump 14 to increase the flow rate "Qeff" of the effluent fluid back to the prescription value or above the prescription value to compensate for the decreasing and to achieve a final dose prescription.

The blood concentration "Cb" of the substance, for instance the plasma water concentration "Cpw", at the inlet 3a of the primary chamber 3 may be also estimated through other criteria. For instance, Cb = k x Ceff_eq, wherein k is an equilibrium constant function of blood parameters of the patient.

According to another embodiment, the blood concentration "Cb" of the substance at the inlet 3a of the primary chamber 3 may be estimated through a recirculation method. According to this recirculation method and referring to Figure 1, the dialysis pump 13 is stopped, the post-infusion pump 24 is stopped, the pre-blood pump 20 and the pre-infusion pump 22 are kept to their prescribed values, the effluent pump 14 is set to balance pre-dilution infusion(s) and deliver patient fluid removal rate and the fluid pump assembly 29 is driven to a maximum flow rate to recirculate the effluent fluid from the outlet 4b of the secondary chamber 4 through the sampling circuit 27 and back to the inlet 4a of the secondary chamber 4.

The concentrate sensor 30 measures periodically the concentration of the substance in the recirculating effluent fluid. A steady state concentration of said substance is extrapolated from the plurality of subsequent measures or the measures are performed until the concentration of said substance becomes stable. The blood concentration Cb_est of the substance at the inlet 3a of the primary chamber 3 is derived from the stable concentration or as the extrapolated steady state. In addition to calculate filter clearance or dialysance "K/D", the blood concentration Cb_est may be used for other monitoring purposes.

According to other examples in which the apparatus carries out Hemodiafiltration (HDF), the concentration of all the supply fluids (in the dialysis line 9, pre-blood pump line 16, pre-infusion line 17, post-infusion line 18) are checked as to compute the solute removal rate "J" through the formula: J = Qeff x Ceff - Σ Qsupi x Csupi; wherein index "i" includes the supply fluids.

The flow rate of supply fluid "Qsup" comprise the flow rate "Qdia" of the dialysis fluid and the flow rate of the replacement fluids in the pre-blood pump line 16, pre-infusion line 17 and post-infusion line 18. The concentrations "Csupi" of the substance to be taken into account is the concentration "Cdia" into the dialysis fluid and the concentrations of the same substance in the replacement fluids of the pre-blood pump line 16, pre-infusion line 17 and post-infusion line 18.

According to another example of monitoring method, the control unit 100 is configured to determine a concentration of a substance in the pure effluent fluid.

To this aim, the control unit 100 may be configured to drive the supply pump while also driving the fluid pump assembly 29 in the second activated condition and acquiring the signal from the concentrate sensor 30 to determine the concentration "Ceff" of the substance into the pure effluent fluid.

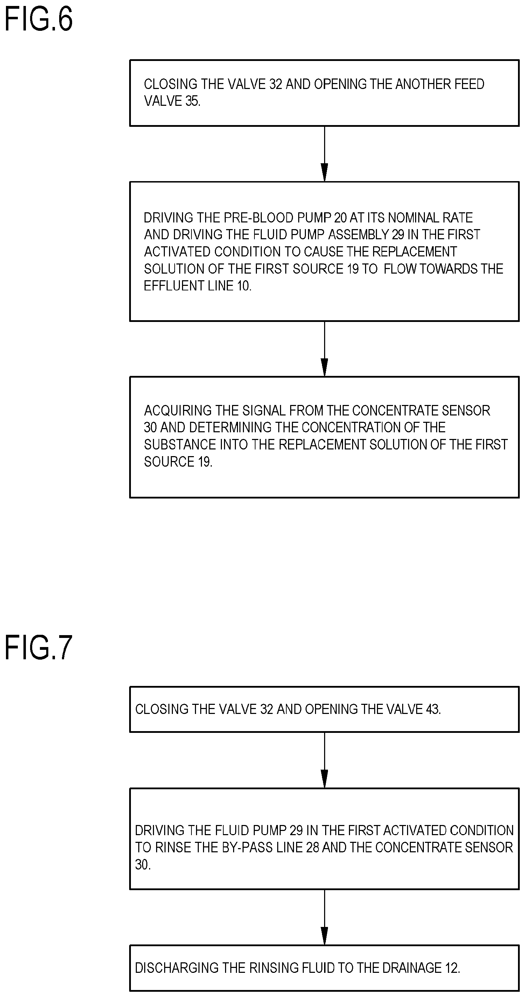

According to an example of monitoring method (Figure 6), in the course of the extracorporeal blood treatment, the control unit 100 is configured to determine a concentration of a substance in the replacement infusion circuit, i.e. in the replacement solution of the first source 19, the second source 21 or the third source 23. This may be carried out as well during system setup as to check for the fluid composition (detection of user errors).

For instance, in order to determine a concentration of a substance in the replacement solution of the first source 19, the control unit 100 closes or keeps closed the feed valve 33 and the further feed valve 37, closes or keeps closed the valve 32, opens the another feed valve 35. The control unit 100 keeps on driving the pre-blood pump 20 at its nominal rate and drives the fluid pump assembly 29 in the first activated condition to cause the replacement solution of the first source 19 to flow towards the effluent line 10 through the feed line 34 and the by-pass line 28. The fluid pump assembly 29 may be stopped as soon as a predefined volume has been pumped through the circuits 34 and 28 to secure that fluid composition in the sensor 30 is fully representative of first source 19. The replacement solution of the first source 19 interacts with the concentrate sensor 30 and the control unit 100 acquires the signal from the concentrate sensor 30 and determines the concentration of the substance into the replacement solution of the first source 19.

Likewise, a concentration of a substance in the replacement solution of the second source 21 may be determined by closing or keeping closed the feed valve 33 and the another feed valve 35, closing or keeping closed the valve 32, opening the further feed valve 37 and driving the fluid pump assembly 29 in the first activated condition thus making the replacement solution of the second source 21 to flow towards the effluent line 10 through the by-pass line 28.

A concentration of a substance **in** the replacement solution of the third source 23 may be determined by closing or keeping closed the another feed valve 35 and the further feed valve 37, closing or keeping closed the valve 32, opening the feed valve 33 and driving the fluid pump assembly 29 in the first activated condition thus making the replacement solution of the third source 23 to flow towards the effluent line 10 through the by-pass line 28.

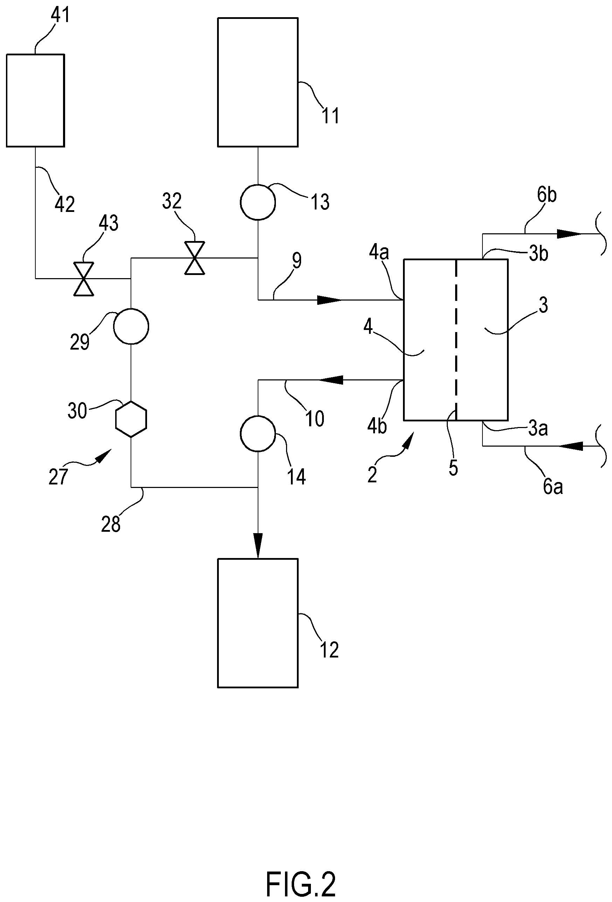

Figure 2 shows a second embodiment of a part of the apparatus according to the invention. In this embodiment, the same reference numerals apply to the same elements of the embodiment of Figure 1. Differently form the first embodiment of Figure 1, in this second embodiment the feed line 31, the other feed line 34 and the further feed line 37 are not depicted but at least one may be present. The concentrate sensor 30 of this embodiment is of a type operating intermittently on the fluid to be tested, like an Ion Selective Electrode sensor (ISE) which needs to be rinsed and calibrated before and after the sample measurement.

Therefore, in addition with respect to the first embodiment of Figure 1, this second embodiment further comprises an auxiliary source 41, e.g. a bag, of an auxiliary fluid and an auxiliary line 42 connecting the auxiliary source 41 to the by-pass line 28 at a location between the dialysis line 9 and the fluid pump assembly 29. An auxiliary valve 43 is operatively active on the auxiliary line 42 and is operated by the control unit 100.

In this second embodiment, the auxiliary fluid in the auxiliary source 41 may be a rinsing fluid and the control unit 100 is configured to rinse the by-pass line 28 and the concentrate sensor 30 before and/or after determining the concentration of the at least one substance. Indeed, the rinsing fluid is configured to rinse the by-pass line 28 while flowing through the by-pass line 28. Rinsing is performed by closing the valve 32, opening the valve 43 and driving the fluid pump assembly 29 in the first activated condition (Figure 7). The circuit design is such that the rinsing fluid is then discharged to the drainage 12 and is not infused in the patient "P", so the rinsing fluid may be a sterile fluid not suitable or not approved for infusion in the patient "P".

In this second embodiment, the auxiliary fluid in the auxiliary source 41 may be a calibration fluid, i.e. a solution of known concentration of the substance(s) to be measured, and the control unit 100 is configured to calibrate the concentrate sensor 30 before and/or after determining the concentration of the at least one substance. The circuit design is such that the calibration fluid is then discharged to the drainage 12 and is not infused in the patient "P", so the calibration fluid may be a sterile fluid not suitable for infusion in the patient "P".

In this second embodiment, the auxiliary fluid in the auxiliary source 41 may be a fluid for quality control and the control unit 100 is configured to control the quality of the concentrate sensor 30 before and/or after determining the concentration of the at least one substance. Also the fluid for quality control may be a sterile fluid not suitable for infusion in the patient "P". The fluids for quality control may be two different solutions that may be representative of the operating limits of the sensor (concentrations next to the lowest and highest concentrations the device is claimed for).

In this second embodiment, since the Ion Selective Electrode sensor (ISE) does not work in a continuous flow and requires static conditions, the control unit 100 stops the fluid pump assembly 29 when the by-pass line 28 is full of the fluid to be measured (e.g. effluent fluid) and the sensor 30 is filled with said fluid and then the concentration of the substance is measured by the ISE sensor 30. Stopping the fluid pump assembly 29 may be required by sensor technology.

In a variant embodiment, the apparatus 1 may be provided with three different bags all connected to the auxiliary line 42 through valves, one bag with the rinsing fluid, one bag with the calibration fluid and one bag with the fluid for quality control.

The auxiliary source/s 41 with the rinsing and/or calibration and/or quality control fluids and the auxiliary line 42 may be part of a (disposable) sampling circuit 27 integrating the concentrate sensor 30.

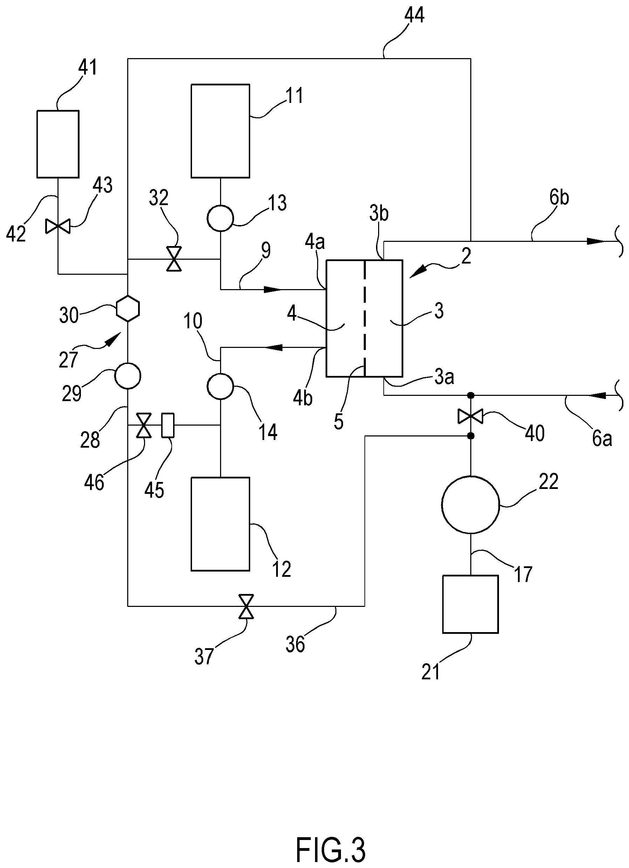

Figure 3 shows a third embodiment of a part of the apparatus according to the invention. In this embodiment, the same reference numerals apply to the same elements of the embodiment of Figure 1.

The third embodiment is like the second embodiment of Figure 2 with the addition of an additional line 44 connecting the blood return line 6b to the by-pass line 28 at a location between the dialysis line 9 and the connection location of the auxiliary line 42. The additional line 44 may have a common section with the post-infusion line 18 (shown in Figure 1). Furthermore, differently from the embodiment of Figures 1 and 2, the positions of the fluid pump assembly 29 and of the concentrate sensor 30 on the by-pass line 28 are swapped. In this embodiment, some calibration fluid of the auxiliary source 41 may be infused to the patient "P". A filter 45 and a further valve 46 are placed on the by-pass line 28 between the effluent line 10 and the fluid pump assembly 29 or the concentrate sensor 12. The filter 45 is intended to prevent possible backflow of germs and may be present or not.

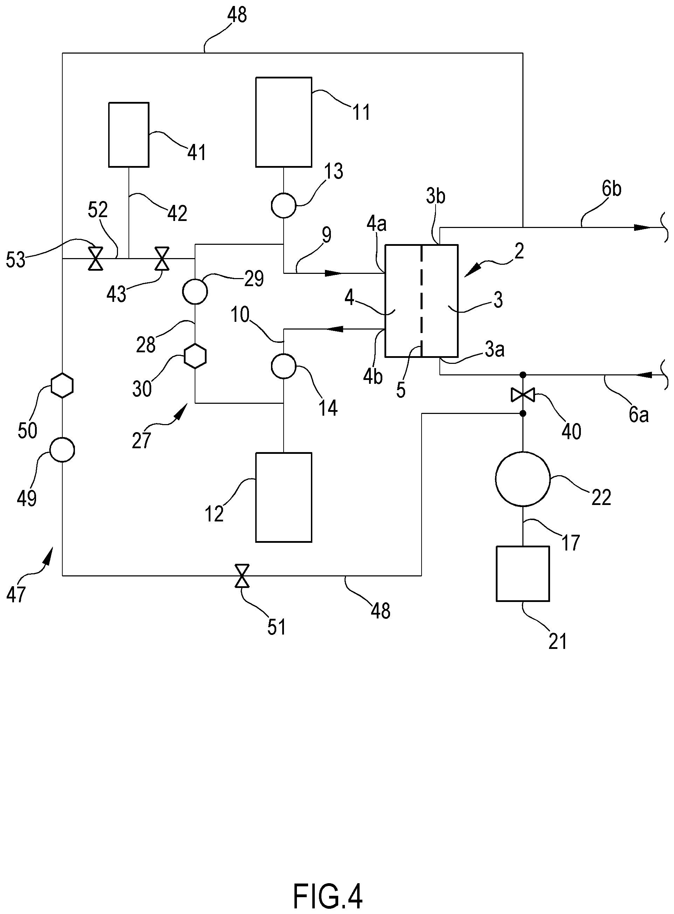

Figure 4 shows a fourth embodiment of a part of the apparatus according to the invention. In this embodiment, the same reference numerals apply to the same elements of the embodiment of Figure 1.

The fourth embodiment is like the first embodiment of Figure 1 with the addition of a monitoring circuit 47 comprising a monitoring line 48 connecting the pre-infusion line 17 to the blood return line 6b. A monitoring pump assembly 49 is active on the monitoring line 48 and a monitoring blood sensor 50 is placed on the monitoring line 48. A monitoring valve 51 is operative on the monitoring line 48 between the pre-infusion line 17 and the monitoring pump assembly 49.

A connecting line 52 provided with a connecting valve 53 connects the auxiliary line 42 to the monitoring line 48 at a location between the blood return line 6b and the monitoring blood sensor 50.

Figure 4 is an example involving two sensors 30, 50, one dedicated to the monitoring of the fluid circuit 8, the other to the monitoring of the extracorporeal blood circuit 6.

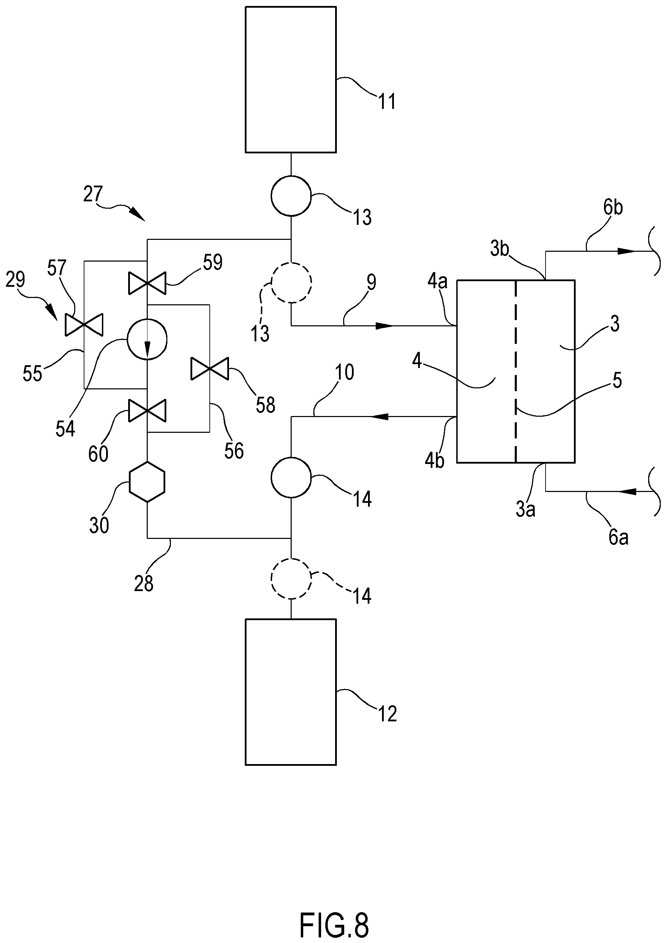

Figure 8 shows a variant embodiment of the fluid pump assembly 29 or of the monitoring pump assembly 49. The fluid pump assembly 29 comprises: a single pump 54 configured to pump always in a same direction; a first by pass branch 55 and a second by-pass branch 56 connecting points of the by-pass line 28 located upstream and downstream of the single pump 54 and assembly valves 57, 58, 59, 60 placed on the first by pass branch 55 on the second by-pass branch 56 and on the by-pass line 28, wherein the control unit 100 is configured to control the assembly valves 57, 58, 59, 60 such to arrange the fluid pump assembly 29 in the first activated condition or in the second activated condition.

In details, in Figure 8, a first assembly valve 57 is operative on the first by pass branch 55, a second assembly valve 58 is operative on the second by pass branch 56. A third assembly valve 59 is operative on the by-pass line 28

between a first connection point of the first by pass branch 55 to the by-pass line 28 and a first connection point of the second by pass branch 56 to the by-pass line 28. The third assembly valve 59 is positioned between the dialysis line 9 and the single pump 54. The first connection point of the first by pass branch 55 is located between the third assembly valve 59 and the dialysis line 9. The first connection point of the second by pass branch 56 is located between the third assembly valve 59 and the single pump 54.

A fourth assembly valve 60 is operative on the by-pass line 28 between a second connection point of the first by pass branch 55 to the by-pass line 28 and a second connection point of the second by pass branch 56 to the by-pass line 28. The second connection point of the first by pass branch 55 is located between the fourth assembly valve 60 and the single pump 54. The second connection point of the second by pass branch 56 is located between the fourth assembly valve 60 and the effluent line 10.

The single pump 54 is configured to pump always in a same direction e.g., from to third assembly valve 59 towards the fourth assembly valve 60. The control unit 100 is configured to drive the fluid pump assembly 29 in the first activated condition by closing the first assembly valve 57 and the second assembly valve 58 and opening the third assembly valve 59 and the fourth assembly valve 60. The control unit 100 is configured to drive the fluid pump assembly 29 in the second activated condition by opening the first assembly valve 57 and the second assembly valve 58 and closing the third assembly valve 59 and the fourth assembly valve 60.

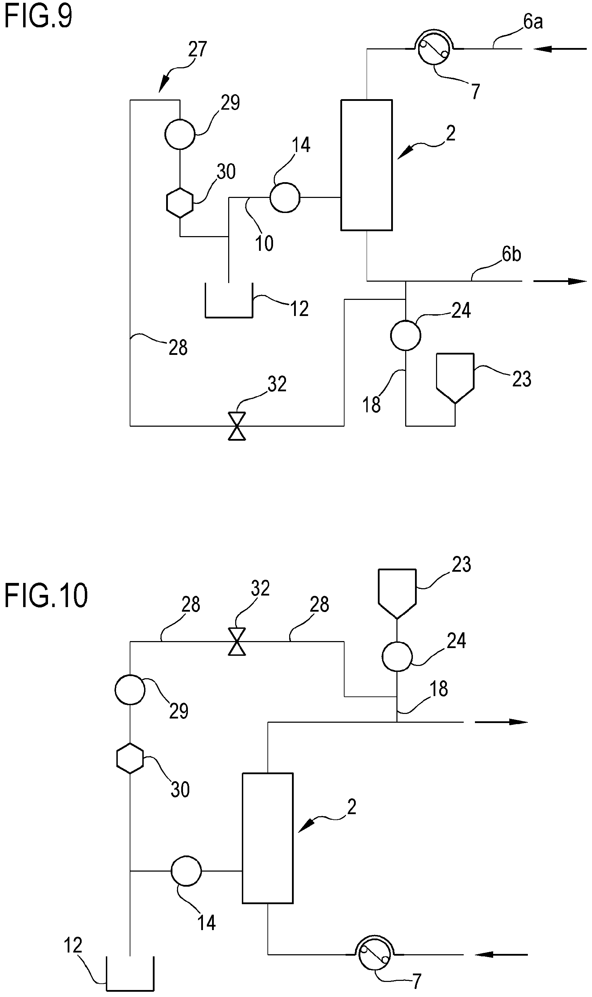

Figures 9 and 10 show further variants of the fluid circuit 8 provided with the sampling circuit 27. Also in these circuits the same reference numerals apply to the same elements of Figure 1.

Figure 9 is a schematic fluid circuit for Therapeutic Plasma Exchange (TPE). The fluid circuit 8 comprises the effluent line 10 with the effluent pump 14 and the replacement line 18 with the source 23 of replacement solution and the pump 24. The by-pass line 28 connects the effluent line 10 to the replacement line 18.

Figure 10 is a schematic fluid circuit in which there is no dialysis line 9. The by-pass line 28 connects the effluent line 10 to the post-infusion line 18.

In further embodiments and irrespectively of the structure of the fluid pump assembly 29, the position of the dialysis pump 13 and of the effluent pump 14 may be different from the position depicted in the drawings. For instance, as shown in dashed line in Figure 8, the dialysis pump 13 may be placed between a branching point of the by-pass line 28 from the dialysis line 9 and the inlet 4a of the secondary chamber 4 and/or the effluent pump 14 may be placed between the drainage 12 and a connection point of the by-pass line 28 to the effluent line 10.

The present monitoring method may be advantageously used for monitoring calcium in connection with cancer dialysis. Indeed, cancer-associated hypercalcemia (CAH) is the most frequent metabolic disorder in cancer patients. CAH is still associated with poor outcome. Hypercalcemia is a condition where there is too much calcium in the blood. It is the most common life-threatening complication of cancer in adults.

Hypercalcemia develops in 10% to 20% of adults with cancer and is a complication that develops in 10% to 20% of patients with cancer. Cancer-associated hypercalcemia often occurs late in the course of solid-tumor development and portends a poor prognosis. The management of hypercalcemia is based on the presence of characteristic symptomatology and the severity of calcium elevation. Patients with hypercalcemia of malignancy are typically symptomatic and have markedly elevated calcium levels.

Dialysis may have a fast impact on calcium reduction, and it remains a viable option to reduce hypercalcemia. Patients with acute hypercalcaemia may be considered for haemodialysis using a very low calcium dialysate (≤1 mmol/L), and monitoring of the blood calcium concentration with the concentration sensor of embodiments of the present description may offer significant advantages for feedback of therapy.

While the invention has been described in connection with what is presently considered to be the most practical and preferred embodiment, it is to be understood that the invention is not to be limited to the disclosed embodiment, but that the invention is defined by the appended claims.

## Claims

**1.** An extracorporeal blood treatment apparatus (1) comprising:

a filtration unit (2) having a primary chamber (3) and a secondary chamber (4) separated by a semipermeable membrane (5);

an extracorporeal blood circuit (6) comprising a blood withdrawal line (6a) connected to an inlet (3a) of the primary chamber (3) and a blood return line (6b) connected to an outlet (3b) of the primary chamber (3), the extracorporeal blood circuit (6) being configured to be connected to a patient (P);

a blood pump (7) active on the extracorporeal blood circuit (6) to pump blood through said extracorporeal blood circuit (6);

a fluid circuit (8) comprising at least one supply line (9, 16, 17, 18) and an effluent line (10), the at least one supply

line (9, 16, 17, 18) being connected to an inlet (4a) of the secondary chamber (4) and/or the extracorporeal blood circuit (6); the effluent line (10) being connected to an outlet (4b) of the secondary chamber (4), the fluid circuit further comprising a source (11, 19, 21, 23) of a supply fluid connected to one end of the at least one supply line (9, 16, 17, 18), a supply pump (13, 20, 22, 24) active on the at least one supply line (9, 16, 17, 18) and an effluent pump (14) active on the effluent line (10);
a sampling circuit (27) comprising a by-pass line (28) connecting the at least one supply line (9, 16, 17, 18) to the effluent line (10);
a fluid pump assembly (29) active on the sampling circuit (27);
a concentrate sensor (30) placed on the by-pass line (28) for monitoring the concentration of at least one substance detectable into the at least one fluid crossing the fluid circuit (8), wherein the at least one fluid is the supply fluid or an effluent fluid flowing out of the secondary chamber (4);
a control unit (100) operatively connected to the concentration sensor (30), the supply pump (13 20, 22, 24), the effluent pump (14) and to the fluid pump assembly (29) and being configured to drive the supply pump and/or the effluent pump to cause the supply fluid to flow into the extracorporeal blood circuit and/or the secondary chamber and to cause the effluent fluid to flow out of the secondary chamber (4), **characterized in that**
the fluid pump assembly (29) is configured for being controlled between a first activated condition, in which the fluid pump assembly (29) causes at least one fluid crossing the fluid circuit (8) to flow through the by-pass line (28) from the at least one supply line (9, 16, 17, 18) to the effluent line (10), and a second activated condition, in which the fluid pump assembly (29) causes at least one fluid crossing the fluid circuit (8) to flow through the by-pass line (28) from the effluent line (10) towards the at least one supply line (9, 16, 17, 18),
the control unit (100) being configured to control the fluid pump assembly (29) both in the first activated condition and in the second activated condition and to receive signals from the concentrate sensor (30) and determine the concentration (Csup, Ceff) of the at least one substance, the control unit being configured to both drive the fluid pump assembly (29) in the first activated condition and acquire the signal from the concentrate sensor (30) to determine the concentration (Csup) of the at least one substance in the supply fluid and drive the fluid pump assembly (29) in the second activated condition and acquire the signal from the concentrate sensor (30) to determine the concentration (Ceff) of the at least one substance in the effluent fluid.

**2.** An extracorporeal blood treatment apparatus according to the previous claim 1, wherein the control unit (100) is configured to estimate a solute removal rate (J) of the at least one substance through the following procedure:

receiving a flow rate (Qeff) of the effluent fluid;
calculating the solute removal rate (J) from the concentration (Ceff) of the at least one substance into the effluent fluid and the flow rate (Qeff) of the effluent fluid;

optionally, the procedure comprising:

receiving a flow rate (Qsup) of the at least one supply fluid;
calculating the solute removal rate (J) also from the concentration (Csup) of the at least one substance into the at least one supply fluid and the flow rate (Qsup) of the at least one supply fluid.

**3.** An extracorporeal blood treatment apparatus according to the previous claim 2, wherein the control unit (100) is configured to estimate a clearance or dialysance (K/D) of the at least one substance through the following procedure: estimating a blood concentration (Cb_est) of the same substance at the inlet (3a) of the primary chamber (3); calculating the clearance or dialysance (K/D) of the at least one substance from the solute removal rate (J) and the estimated blood concentration (Cb_est) of the same substance at the inlet (3a) of the primary chamber (3).

**4.** An extracorporeal blood treatment apparatus according to the previous claim 3, wherein estimating a blood concentration (Cb_est) of the same substance at the inlet (3a) of the primary chamber (3) comprises: securing equilibrium between the concentration (Ceff_eq) of the at least one substance into the effluent fluid and a blood concentration (Cb) of the same substance at the inlet (3a) of the primary chamber (3) such that the concentration (Ceff) of the at least one substance into the effluent fluid equals or is proportional to the blood concentration (Cb) of the at least one substance at the inlet (3a) of the primary chamber (3); wherein securing equilibrium is achieved by changing the flow rate (Qsup) of the at least one supply fluid and/or the flow rate (Qeff) of the effluent fluid and/or a blood flow rate (Qb) set for the extracorporeal blood treatment.

**5.** An extracorporeal blood treatment apparatus according to the previous claim 4, wherein securing equilibrium is achieved by waiting for a stabilization time (t) after changing the flow rate (Qsup) of the at least one supply fluid and/or

the flow rate (Qeff) of the effluent fluid and/or a blood flow rate (Qb).

**6.** An extracorporeal blood treatment apparatus according to any of the previous claims 4 or 5, wherein, after estimating the blood concentration (Cb_est), the control unit is configured to, or the method provides for, moving back the flow rate Qsup of the at least one supply fluid and/or the flow rate Qeff of the effluent fluid and/or a blood flow rate Qb to the prescription values.

**7.** An extracorporeal blood treatment apparatus according to the previous claim 3, wherein estimating a blood concentration (Cb_est) of the same substance at the inlet (3a) of the primary chamber (3) comprises: recirculating at least part of the effluent fluid from the outlet (4b) of the secondary chamber (4) through the sampling circuit (27) and back to the inlet (4a) of the secondary chamber (4); measuring the concentration of said substance, optionally periodically, in the recirculating effluent fluid; waiting until the concentration of said substance becomes stable or extrapolating a steady state concentration of said substance; the estimated blood concentration (Cb_est) of the substance at the inlet (3a) of the primary chamber (3) being the stable concentration or the extrapolated steady state; optionally setting valves in proper states to route the effluent fluid.

**8.** An extracorporeal blood treatment apparatus according to any of the previous claims 1 to 7, wherein the at least one supply line (9, 16, 17, 18) comprises:

a dialysis line (9) connected to the inlet of the secondary chamber (4), a dialysis pump (13) active on the dialysis line (9), a source (11) of dialysis fluid connected to one end of the dialysis line (9);
and/or
the at least one supply line (9, 16, 17, 18) comprising:

at least one replacement infusion line (16, 17, 18) connected to the extracorporeal blood circuit, upstream or downstream of the filtration unit (2), or directly to the patient (P);
at least one replacement infusion pump (20, 22, 24) active on the at least one replacement infusion line (16, 17, 18);
at least one source (19, 21, 23) of replacement solution connected to one end of the at least one replacement infusion line (16, 17, 18);
wherein the by-pass line (28) connects the at least one replacement infusion line (16, 17, 18) to the effluent line (10); or
wherein the by-pass line (28) connects the dialysis line (9) to the effluent line (10) and said by-pass line (28) further comprises a feed line (31, 34, 36) connecting the at least one replacement infusion line (16, 17, 18) to the by-pass line (28) at a location between the dialysis line (9) and the concentrate sensor (30).

**9.** An extracorporeal blood treatment apparatus according to claim 1 or 2, wherein the fluid circuit (8) further comprises an auxiliary source (41) of an auxiliary fluid connected to the by-pass line (28) at a location between the at least one supply line (9, 16, 17, 18) and the concentrate sensor (30); wherein the control unit (100) is configured to:
drive the fluid pump assembly (29), and optionally to control valves, to cause the auxiliary fluid to flow through the by-pass line (28).

**10.** An extracorporeal blood treatment apparatus according to the previous claim 9, wherein the auxiliary fluid is a calibration fluid and the control unit (100) is configured to calibrate the concentrate sensor (100) while said auxiliary fluid flows through the by-pass line (28).

**11.** An extracorporeal blood treatment apparatus according to any to claims 1 to 10, wherein the control unit (100) is configured to control the extracorporeal blood treatment apparatus to perform an extracorporeal blood treatment on the patient (P) and is configured determine the concentration of the at least one substance or to monitor the at least one substance during the extracorporeal blood treatment.

**12.** An extracorporeal blood treatment apparatus according to any of the previous claims 1 to 11, wherein the fluid pump assembly (29) comprises a single reversible pump acting on the by-pass line (28).

**13.** An extracorporeal blood treatment apparatus according to any of the previous claims 1 to 11, wherein the fluid pump assembly (29) comprises: a single pump (54) configured to pump always in a same direction; a first by pass branch (55) and a second by-pass branch (56) connecting points of the by-pass line (28) located upstream and downstream of the single pump (54) and assembly valves (57, 58, 59, 60) placed on the first by pass branch (55) on the second by-

pass branch (56) and on the by-pass line (28), wherein the control unit (100) is configured to control the assembly valves (57, 58, 59, 60) such to arrange the fluid pump assembly (29) in the first activated condition or in the second activated condition.

**14.** An extracorporeal blood treatment apparatus according any of the previous claims 1 to 13, wherein the sampling circuit (27) is disposable or is part of a disposable set or is connectable to the disposable set, the disposable set comprising the extracorporeal blood circuit (6) and the fluid circuit (8).

**15.** An extracorporeal blood treatment apparatus according any of the previous claims 1 to 14, wherein the at least one substance may be urea, creatinine, uric acid, glucose, electrolytes, lactate.

**Patentansprüche**

**1.** Extrakorporales Blutbehandlungsgerät (1), umfassend:

eine Filtrationseinheit (2) mit einer Primärkammer (3) und einer Sekundärkammer (4), die durch eine semipermeable Membran (5) getrennt sind;
einen extrakorporalen Blutkreislauf (6), umfassend eine Blutentnahmeleitung (6a), die mit einem Einlass (3a) der Primärkammer (3) verbunden ist, und eine Blutrückgabeleitung (6b), die mit einem Auslass (3b) der Primärkammer (3) verbunden ist, wobei der extrakorporale Blutkreislauf (6) zur Verbindung mit einem Patienten (P) ausgelegt ist;
eine Blutpumpe (7), die an dem extrakorporalen Blutkreislauf (6) aktiv ist, um Blut durch den extrakorporalen Blutkreislauf (6) zu pumpen;
einen Fluidkreislauf (8), umfassend mindestens eine Versorgungsleitung (9, 16, 17, 18) und eine Abflussleitung (10), wobei die mindestens eine Versorgungsleitung (9, 16, 17, 18) mit einem Einlass (4a) der Sekundärkammer (4) und/oder dem extrakorporalen Blutkreislauf (6) verbunden ist; wobei die Abflussleitung (10) mit einem Auslass (4b) der Sekundärkammer (4) verbunden ist, wobei der Fluidkreislauf ferner eine Quelle (11, 19, 21, 23) eines Versorgungsfluids, die mit einem Ende der mindestens einen Versorgungsleitung (9, 16, 17, 18) verbunden ist, eine Versorgungspumpe (13, 20, 22, 24), die an der mindestens einen Versorgungsleitung (9, 16, 17, 18) aktiv ist, und eine Abflusspumpe (14) umfasst, die an der Abflussleitung (10) aktiv ist;
einen Probenahmekreislauf (27), umfassend eine Umgehungsleitung (28), die die mindestens eine Versorgungsleitung (9, 16, 17, 18) mit der Abflussleitung (10) verbindet;
eine Fluidpumpenanordnung (29), die an dem Probenahmekreislauf (27) aktiv ist;
einen Konzentrationssensor (30), der an der Umgehungsleitung (28) platziert ist, um die Konzentration mindestens einer Substanz zu überwachen, die in dem mindestens einen den Fluidkreislauf (8) durchquerenden Fluid detektierbar ist, wobei das mindestens eine Fluid das Versorgungsfluid oder ein Abflussfluid ist, das aus der Sekundärkammer (4) fließt;
eine Steuereinheit (100), die operativ mit dem Konzentrationssensor (30), der Versorgungspumpe (13, 20, 22, 24), der Abflusspumpe (14) und der Fluidpumpenanordnung (29) verbunden ist und dazu ausgelegt ist, die Versorgungspumpe und/oder die Abflusspumpe anzutreiben, um zu bewirken, dass das Versorgungsfluid in den extrakorporalen Blutkreislauf und/oder die Sekundärkammer fließt, und um zu bewirken, dass das Abflussfluid aus der Sekundärkammer (4) fließt, **dadurch gekennzeichnet, dass**
die Fluidpumpenanordnung (29) dazu ausgelegt ist, zwischen einem ersten aktivierten Zustand, in dem die Fluidpumpenanordnung (29) bewirkt, dass mindestens ein Fluid, das den Fluidkreislauf (8) durchquert, durch die Umgehungsleitung (28) von der mindestens einen Versorgungsleitung (9, 16, 17, 18) zu der Abflussleitung (10) fließt, und einem zweiten aktivierten Zustand gesteuert zu werden, in dem die Fluidpumpenanordnung (29) bewirkt, dass mindestens ein Fluid, das den Fluidkreislauf (8) durchquert, durch die Umgehungsleitung (28) von der Abflussleitung (10) zu der mindestens einen Versorgungsleitung (9, 16, 17, 18) fließt,
wobei die Steuereinheit (100) dazu ausgelegt ist, die Fluidpumpenanordnung (29) sowohl in dem ersten aktivierten Zustand als auch in dem zweiten aktivierten Zustand zu steuern und Signale von dem Konzentrationssensor (30) zu empfangen und die Konzentration (Csup, Ceff) der mindestens einen Substanz zu bestimmen, wobei die Steuereinheit dazu ausgelegt ist, sowohl die Fluidpumpenanordnung (29) in dem ersten aktivierten Zustand anzutreiben und das Signal von dem Konzentrationssensor (30) zu erlangen, um die Konzentration (Csup) der mindestens einen Substanz in dem Versorgungsfluid zu bestimmen, als auch die Fluidpumpenanordnung (29) in dem zweiten aktivierten Zustand anzutreiben und das Signal von dem Konzentrationssensor (30) zu erlangen, um die Konzentration (Ceff) der mindestens einen Substanz in dem Abflussfluid zu bestimmen.

2. Extrakorporales Blutbehandlungsgerät nach dem vorhergehenden Anspruch 1, wobei die Steuereinheit (100) dazu ausgelegt ist, eine Entfernungsrate des gelösten Stoffs (J) der mindestens einen Substanz durch die folgende Vorgehensweise zu schätzen:

   Empfangen einer Flussrate (Qeff) des Abflussfluids;
   Berechnen der Entfernungsrate des gelösten Stoffs (J) aus der Konzentration (Ceff) der mindestens einen Substanz in dem Abflussfluid und der Flussrate (Qeff) des Abflussfluids;
   wobei die Vorgehensweise gegebenenfalls umfasst:

      Empfangen einer Flussrate (Qsup) des mindestens einen Versorgungsfluids;
      Berechnen der Entfernungsrate des gelösten Stoffs (J) auch aus der Konzentration (Csup) der mindestens einen Substanz in dem mindestens einen Versorgungsfluid und der Flussrate (Qsup) des mindestens einen Versorgungsfluids.

3. Extrakorporales Blutbehandlungsgerät nach dem vorhergehenden Anspruch 2, wobei die Steuereinheit (100) dazu ausgelegt ist, eine Clearance oder Dialysance (K/D) der mindestens einen Substanz durch die folgende Vorgehensweise zu schätzen: Schätzen einer Blutkonzentration (Cb_est) derselben Substanz am Einlass (3a) der Primärkammer (3); Berechnen der Clearance oder Dialysance (K/D) der mindestens einen Substanz aus der Entfernungsrate des gelösten Stoffs (J) und der geschätzten Blutkonzentration (Cb_est) derselben Substanz am Einlass (3a) der Primärkammer (3).

4. Extrakorporales Blutbehandlungsgerät nach dem vorhergehenden Anspruch 3, wobei Schätzen einer Blutkonzentration (Cb_est) derselben Substanz am Einlass (3a) der Primärkammer (3) umfasst: Sicherstellen eines Gleichgewichts zwischen der Konzentration (Ceff_eq) der mindestens einen Substanz in dem Abflussfluid und einer Blutkonzentration (Cb) derselben Substanz am Einlass (3a) der Primärkammer (3), so dass die Konzentration (Ceff) der mindestens einen Substanz in dem Abflussfluid gleich oder proportional zu der Blutkonzentration (Cb) der mindestens einen Substanz am Einlass (3a) der Primärkammer (3) ist; wobei Sicherstellen eines Gleichgewichts durch Ändern der Flussrate (Qsup) des mindestens einen Versorgungsfluids und/oder der Flussrate (Qeff) des Abflussfluids und/oder einer für die extrakorporale Blutbehandlung eingestellten Blutflussrate (Qb) erreicht wird.

5. Extrakorporales Blutbehandlungsgerät nach dem vorhergehenden Anspruch 4, wobei Sicherstellen eines Gleichgewichts durch Warten für eine Stabilisierungszeit (t) nach Ändern der Flussrate (Qsup) des mindestens einen Versorgungsfluids und/oder der Flussrate (Qeff) des Abflussfluids und/oder einer Blutflussrate (Qb) erreicht wird.

6. Extrakorporales Blutbehandlungsgerät nach einem der vorhergehenden Ansprüche 4 oder 5, wobei die Steuereinheit dazu ausgelegt ist, nach Schätzen der Blutkonzentration (Cb_est) die Flussrate Qsup des mindestens einen Versorgungsfluids und/oder die Flussrate Qeff des Abflussfluids und/oder eine Blutflussrate Qb auf die Verordnungswerte zurückzuführen, oder wobei das Verfahren ein entsprechendes Zurückführen vorsieht.

7. Extrakorporales Blutbehandlungsgerät nach dem vorhergehenden Anspruch 3, wobei Schätzen einer Blutkonzentration (Cb_est) derselben Substanz am Einlass (3a) der Primärkammer (3) umfasst: Rezirkulieren mindestens eines Teils des Abflussfluids aus dem Auslass (4b) der Sekundärkammer (4) durch den Probenahmekreislauf (27) und zurück zu dem Einlass (4a) der Sekundärkammer (4); gegebenenfalls periodisches Messen der Konzentration der Substanz in dem Rezirkulationsabflussfluid; Warten, bis die Konzentration der Substanz stabil wird, oder Extrapolieren einer stationären Konzentration der Substanz; wobei die geschätzte Blutkonzentration (Cb_est) der Substanz am Einlass (3a) der Primärkammer (3) die stabile Konzentration oder die extrapolierte stationäre Konzentration ist; gegebenenfalls Einstellen von Ventilen in die richtigen Zustände, um das Abflussfluid zu führen.

8. Extrakorporales Blutbehandlungsgerät nach einem der vorhergehenden Ansprüche 1 bis 7, wobei die mindestens eine Versorgungsleitung (9, 16, 17, 18) umfasst:

   eine Dialyseleitung (9), die mit dem Einlass der Sekundärkammer (4) verbunden ist, eine Dialysepumpe (13), die an der Dialyseleitung (9) aktiv ist, eine Quelle (11) für Dialysefluid, die mit einem Ende der Dialyseleitung (9) verbunden ist;
   und/oder
   wobei die mindestens eine Versorgungsleitung (9, 16, 17, 18) umfasst:

      mindestens eine Ersatzinfusionsleitung (16, 17, 18), die mit dem extrakorporalen Blutkreislauf stromauf-

wärts oder stromabwärts der Filtrationseinheit (2) oder direkt mit dem Patienten (P) verbunden ist;
mindestens eine Ersatzinfusionspumpe (20, 22, 24), die an der mindestens einen Ersatzinfusionsleitung (16, 17, 18) aktiv ist;
mindestens eine Quelle (19, 21, 23) von Ersatzlösung, die mit einem Ende der mindestens einen Ersatzinfusionsleitung (16, 17, 18) verbunden ist;
wobei die Umgehungsleitung (28) die mindestens eine Ersatzinfusionsleitung (16, 17, 18) mit der Abflussleitung (10) verbindet; oder
wobei die Umgehungsleitung (28) die Dialyseleitung (9) mit der Abflussleitung (10) verbindet, und die Umgehungsleitung (28) ferner eine Speiseleitung (31, 34, 36) umfasst, die die mindestens eine Ersatzinfusionsleitung (16, 17, 18) mit der Umgehungsleitung (28) an einer Stelle zwischen der Dialyseleitung (9) und dem Konzentrationssensor (30) verbindet.

**9.** Extrakorporales Blutbehandlungsgerät nach Anspruch 1 oder 2, wobei der Fluidkreislauf (8) ferner eine Hilfsquelle (41) eines Hilfsfluids umfasst, die mit der Umgehungsleitung (28) an einer Stelle zwischen der mindestens einen Versorgungsleitung (9, 16, 17, 18) und dem Konzentrationssensor (30) verbunden ist; wobei die Steuereinheit (100) ausgelegt ist zum:
Antreiben der Fluidpumpenanordnung (29) und gegebenenfalls Steuern von Ventilen, um zu bewirken, dass das Hilfsfluid durch die Umgehungsleitung (28) fließt.

**10.** Extrakorporales Blutbehandlungsgerät nach dem vorhergehenden Anspruch 9, wobei das Hilfsfluid ein Kalibrierfluid ist und die Steuereinheit (100) dazu ausgelegt ist, den Konzentrationssensor (100) zu kalibrieren, während das Hilfsfluid durch die Umgehungsleitung (28) fließt.

**11.** Extrakorporales Blutbehandlungsgerät nach einem der Ansprüche 1 bis 10, wobei die Steuereinheit (100) dazu ausgelegt ist, das extrakorporale Blutbehandlungsgerät zu steuern, um eine extrakorporale Blutbehandlung an dem Patienten (P) durchzuführen, und dazu ausgelegt ist, die Konzentration der mindestens einen Substanz zu bestimmen oder die mindestens eine Substanz während der extrakorporalen Blutbehandlung zu überwachen.

**12.** Extrakorporales Blutbehandlungsgerät nach einem der vorhergehenden Ansprüche 1 bis 11, wobei die Fluidpumpenanordnung (29) eine einzige Reversierpumpe umfasst, die auf die Umgehungsleitung (28) wirkt.

**13.** Extrakorporales Blutbehandlungsgerät nach einem der vorhergehenden Ansprüche 1 bis 11, wobei die Fluidpumpenanordnung (29) umfasst: eine einzige Pumpe (54), die dazu ausgelegt ist, immer in eine gleiche Richtung zu pumpen; einen ersten Umgehungszweig (55) und einen zweiten Umgehungszweig (56), die Punkte der Umgehungsleitung (28) verbinden, die sich stromaufwärts und stromabwärts der einzelnen Pumpe (54) befinden, und Anordnungsventile (57, 58, 59, 60), die an dem ersten Umgehungszweig (55), an dem zweiten Umgehungszweig (56) und an der Umgehungsleitung (28) platziert sind, wobei die Steuereinheit (100) dazu ausgelegt ist, die Anordnungsventile (57, 58, 59, 60) so zu steuern, dass die Fluidpumpenanordnung (29) in dem ersten aktivierten Zustand oder in dem zweiten aktivierten Zustand angeordnet wird.

**14.** Extrakorporales Blutbehandlungsgerät nach einem der vorhergehenden Ansprüche 1 bis 13, wobei der Probenahmekreislauf (27) wegwerfbar ist oder Teil eines Wegwerfsatzes ist oder mit dem Wegwerfsatz verbindbar ist, wobei der Wegwerfsatz den extrakorporalen Blutkreislauf (6) und den Fluidkreislauf (8) umfasst.

**15.** Extrakorporales Blutbehandlungsgerät nach einem der vorhergehenden Ansprüche 1 bis 14, wobei die mindestens eine Substanz Harnstoff, Kreatinin, Harnsäure, Glucose, Elektrolyte, Laktat sein kann.

## Revendications

**1.** Appareil de traitement sanguin extracorporel (1) comprenant :

une unité de filtration (2) ayant une chambre primaire (3) et une chambre secondaire (4) séparées par une membrane semi-perméable (5) ;
un circuit sanguin extracorporel (6) comprenant un conduit de prélèvement de sang (6a) relié à une entrée (3a) de la chambre primaire (3), et un conduit de retour de sang (6b) relié à une sortie (3b) de la chambre primaire (3), le circuit sanguin extracorporel (6) étant apte à être relié à un patient (P) ;
une pompe à sang (7) active sur le circuit sanguin extracorporel (6) pour pomper le sang à travers ledit circuit

sanguin extracorporel (6) ;
un circuit de fluide (8) comprenant au moins un conduit d'alimentation (9, 16, 17, 18) et un conduit d'effluent (10), l'au moins un conduit d'alimentation (9, 16, 17, 18) étant relié à une entrée (4a) de la chambre secondaire (4) et/ou au circuit sanguin extracorporel (6) ; le conduit d'effluent (10) étant relié à une sortie (4b) de la chambre secondaire (4), le circuit de fluide comprenant en outre une source (11, 19, 21, 23) d'un fluide d'alimentation reliée à une extrémité de l'au moins un conduit d'alimentation (9, 16, 17, 18), une pompe d'alimentation (13, 20, 22, 24) active sur l'au moins un conduit d'alimentation (9, 16, 17, 18) et une pompe à effluent (14) active sur le conduit d'effluent (10) ;
un circuit d'échantillonnage (27) comprenant un conduit de dérivation (28) reliant l'au moins un conduit d'alimentation (9, 16, 17, 18) au conduit d'effluent (10) ;
un ensemble de pompe à fluide (29) actif sur le circuit d'échantillonnage (27) ;
un capteur de concentration (30) placé sur le conduit de dérivation (28) pour surveiller la concentration d'au moins une substance détectable dans l'au moins un fluide traversant le circuit de fluide (8), l'au moins un fluide étant le fluide d'alimentation ou un fluide effluent s'écoulant hors de la chambre secondaire (4) ;
une unité de commande (100) reliée fonctionnellement au capteur de concentration (30), à la pompe d'alimentation (13, 20, 22, 24), à la pompe à effluent (14) et à l'ensemble de pompe à fluide (29) et étant apte à entraîner la pompe d'alimentation et/ou la pompe à effluent pour amener le fluide d'alimentation à s'écouler dans le circuit sanguin extracorporel et/ou la chambre secondaire et pour amener le fluide effluent à s'écouler hors de la chambre secondaire (4), **caractérisé en ce que**
l'ensemble de pompe à fluide (29) est apte à être commandé entre un premier état activé, dans lequel l'ensemble de pompe à fluide (29) amène au moins un fluide traversant le circuit de fluide (8) à s'écouler à travers le conduit de dérivation (28) depuis l'au moins un conduit d'alimentation (9, 16, 17, 18) vers le conduit d'effluent (10), et un second état activé, dans lequel l'ensemble de pompe à fluide (29) amène au moins un fluide traversant le circuit de fluide (8) à s'écouler à travers le conduit de dérivation (28) depuis le conduit d'effluent (10) vers l'au moins un conduit d'alimentation (9, 16, 17, 18),
l'unité de commande (100) étant apte à commander l'ensemble de pompe à fluide (29) à la fois dans le premier état activé et dans le second état activé et à recevoir des signaux du capteur de concentration (30) et à déterminer la concentration (Csup, Ceff) de l'au moins une substance, l'unité de commande étant apte à la fois à commander l'ensemble de pompe à fluide (29) dans le premier état activé et à acquérir le signal du capteur de concentration (30) pour déterminer la concentration (Csup) de l'au moins une substance dans le fluide d'alimentation et à commander l'ensemble de pompe à fluide (29) dans le second état activé et à acquérir le signal du capteur de concentration (30) pour déterminer la concentration (Ceff) de l'au moins une substance dans le fluide effluent.

**2.** Appareil de traitement sanguin extracorporel selon la revendication 1, l'unité de commande (100) étant apte à estimer un taux d'élimination de soluté (J) de l'au moins une substance par la procédure suivante :

la réception d'un débit (Qeff) du fluide effluent ;
le calcul du taux d'élimination de soluté (J) à partir de la concentration (Ceff) de l'au moins une substance dans le fluide effluent et du débit (Qeff) du fluide effluent ;
éventuellement, la procédure comprenant :

la réception d'un débit (Qsup) de l'au moins un fluide d'alimentation ;
le calcul du taux d'élimination de soluté (J) également à partir de la concentration (Csup) de l'au moins une substance dans l'au moins un fluide d'alimentation et du débit (Qsup) de l'au moins un fluide d'alimentation.

**3.** Appareil de traitement sanguin extracorporel selon la revendication 2, l'unité de commande (100) étant apte à estimer une clairance ou une dialysance (K/D) de l'au moins une substance par la procédure suivante : l'estimation d'une concentration sanguine (Cb_est) de la même substance à l'entrée (3a) de la chambre primaire (3) ; le calcul de la clairance ou de la dialysance (K/D) de l'au moins une substance à partir du taux d'élimination de soluté (J) et de la concentration sanguine estimée (Cb_est) de la même substance à l'entrée (3a) de la chambre primaire (3).

**4.** Appareil de traitement sanguin extracorporel selon la revendication 3, l'estimation d'une concentration sanguine (Cb_est) de la même substance à l'entrée (3a) de la chambre primaire (3) comprenant : l'obtention d'un équilibre entre la concentration (Ceff_eq) de l'au moins une substance dans le fluide effluent et une concentration sanguine (Cb) de la même substance à l'entrée (3a) de la chambre primaire (3) de sorte que la concentration (Ceff) de l'au moins une substance dans le fluide effluent soit égale ou proportionnelle à la concentration sanguine (Cb) de l'au moins une substance à l'entrée (3a) de la chambre primaire (3) ; l'obtention de l'équilibre étant réalisée en modifiant le débit (Qsup) de l'au moins un fluide d'alimentation et/ou le débit (Qeff) du fluide effluent et/ou un débit sanguin (Qb) réglé

pour le traitement sanguin extracorporel.

**5.** Appareil de traitement sanguin extracorporel selon la revendication 4, l'obtention de l'équilibre étant réalisée par l'attente d'un temps de stabilisation (t) après la modification du débit (Qsup) de l'au moins un fluide d'alimentation et/ou du débit (Qeff) du fluide effluent et/ou d'un débit sanguin (Qb).

**6.** Appareil de traitement sanguin extracorporel selon l'une des revendications précédentes 4 et 5, après l'estimation de la concentration sanguine (Cb_est), l'unité de commande étant apte à, ou le procédé prévoit de, ramener le débit Qsup de l'au moins un fluide d'alimentation et/ou le débit Qeff du fluide effluent et/ou un débit sanguin Qb aux valeurs de prescription.

**7.** Appareil de traitement sanguin extracorporel selon la revendication 3, l'estimation d'une concentration sanguine (Cb_est) de la même substance à l'entrée (3a) de la chambre primaire (3) comprenant : la recirculation d'au moins une partie du fluide effluent depuis la sortie (4b) de la chambre secondaire (4) à travers le circuit d'échantillonnage (27) et de retour vers l'entrée (4a) de la chambre secondaire (4) ; la mesure de la concentration de ladite substance, éventuellement périodiquement, dans le fluide effluent en recirculation ; l'attente jusqu'à ce que la concentration de ladite substance devienne stable ou l'extrapolation d'une concentration à l'état stationnaire de ladite substance ; la concentration sanguine estimée (Cb_est) de la substance à l'entrée (3a) de la chambre primaire (3) étant la concentration stable ou l'état stationnaire extrapolé ; éventuellement le réglage de vannes dans des états appropriés pour acheminer le fluide effluent.

**8.** Appareil de traitement sanguin extracorporel selon l'une quelconque des revendications précédentes 1 à 7, l'au moins un conduit d'alimentation (9, 16, 17, 18) comprenant :

un conduit de dialyse (9) relié à l'entrée de la chambre secondaire (4), une pompe de dialyse (13) active sur le conduit de dialyse (9), une source (11) de fluide de dialyse reliée à une extrémité du conduit de dialyse (9) ; et/ou
l'au moins un conduit d'alimentation (9, 16, 17, 18) comprenant :

au moins un conduit de perfusion de remplacement (16, 17, 18) relié au circuit sanguin extracorporel, en amont ou en aval de l'unité de filtration (2), ou directement au patient (P) ;
au moins une pompe de perfusion de remplacement (20, 22, 24) active sur l'au moins un conduit de perfusion de remplacement (16, 17, 18) ;
au moins une source (19, 21, 23) de solution de remplacement reliée à une extrémité de l'au moins un conduit de perfusion de remplacement (16, 17, 18) ;
le conduit de dérivation (28) reliant l'au moins un conduit de perfusion de remplacement (16, 17, 18) au conduit d'effluent (10) ; ou
le conduit de dérivation (28) reliant le conduit de dialyse (9) au conduit d'effluent (10) et ledit conduit de dérivation (28) comprenant en outre un conduit d'amenée (31, 34, 36) reliant l'au moins un conduit de perfusion de remplacement (16, 17, 18) au conduit de dérivation (28) à un emplacement situé entre le conduit de dialyse (9) et le capteur de concentration (30).

**9.** Appareil de traitement sanguin extracorporel selon la revendication 1 ou 2, le circuit de fluide (8) comprenant en outre une source auxiliaire (41) d'un fluide auxiliaire reliée au conduit de dérivation (28) à un emplacement situé entre l'au moins un conduit d'alimentation (9, 16, 17, 18) et le capteur de concentration (30) ; l'unité de commande (100) étant apte à :
commander l'ensemble de pompe à fluide (29), et éventuellement à commander des vannes, afin d'amener le fluide auxiliaire à s'écouler à travers le conduit de dérivation (28).

**10.** Appareil de traitement sanguin extracorporel selon la revendication 9, le fluide auxiliaire étant un fluide d'étalonnage et l'unité de commande (100) étant apte à étalonner le capteur de concentration (100) pendant que ledit fluide auxiliaire s'écoule à travers le conduit de dérivation (28).

**11.** Appareil de traitement sanguin extracorporel selon l'une quelconque des revendications 1 à 10, l'unité de commande (100) étant apte à commander l'appareil de traitement sanguin extracorporel afin d'effectuer un traitement sanguin extracorporel sur le patient (P) et étant apte à déterminer la concentration de l'au moins une substance ou à surveiller l'au moins une substance pendant le traitement sanguin extracorporel.

**12.** Appareil de traitement sanguin extracorporel selon l'une quelconque des revendications précédentes 1 à 11, l'ensemble de pompe à fluide (29) comprenant une pompe réversible unique agissant sur le conduit de dérivation (28).

**13.** Appareil de traitement sanguin extracorporel selon l'une quelconque des revendications précédentes 1 à 11, l'ensemble de pompe à fluide (29) comprenant : une pompe unique (54) apte à pomper toujours dans une même direction ; une première branche de dérivation (55) et une seconde branche de dérivation (56) reliant des points du conduit de dérivation (28) situés en amont et en aval de la pompe unique (54) et des vannes d'assemblage (57, 58, 59, 60) placées sur la première branche de dérivation (55), sur la seconde branche de dérivation (56) et sur le conduit de dérivation (28), l'unité de commande (100) étant apte à commander les vannes d'assemblage (57, 58, 59, 60) de manière à disposer l'ensemble de pompe à fluide (29) dans le premier état activé ou dans le second état activé.

**14.** Appareil de traitement sanguin extracorporel selon l'une quelconque des revendications précédentes 1 à 13, le circuit d'échantillonnage (27) étant jetable ou faisant partie d'un ensemble jetable ou étant apte à être relié à l'ensemble jetable, l'ensemble jetable comprenant le circuit sanguin extracorporel (6) et le circuit de fluide (8).

**15.** Appareil de traitement sanguin extracorporel selon l'une quelconque des revendications précédentes 1 à 14, l'au moins une substance pouvant être l'urée, la créatinine, l'acide urique, le glucose, des électrolytes, le lactate.

FIG.1
P
1
2
3
3a
3b
4
4a
4b
5
6
6a
6b
7
8
9
10
11
12
13
14
15
16
17
18
19
20
21
22
23
24
25
26
27
28
29
30
31
32
33
34
35
36
37
38
39
40
100
110

41
11
42
32
13
6b
43
9
4a
3b
3
29
4
30
10
4b
3a
27
14
2
5
6a
28
12

FIG.2

44
41
11
43
32
13
42
3b
6b
9
4a
2
30
10
4
3
27
29
4b
3a
5
14
40
6a
28
46
45
22
12
17
37
36
21

FIG.3

48
11
41
13
3b
6b
52
42
4a
9
2
53
43
29
4
3
10
28
3a
30
4b
5
6a
50
14
40
27
49
22
12
17
47
51
48
21

FIG.4

FIG.5

DRIVING THE FLUID PUMP ASSEMBLY 29 IN THE FIRST ACTIVATED CONDITION AND ACQUIRING THE SIGNAL FROM THE CONCENTRATE SENSOR 30 TO DETERMINE THE CONCENTRATION "Cdia" OF THE SUBSTANCE INTO THE DIALYSIS FLUID.

↓

DRIVING THE FLUID PUMP 29 IN THE SECOND ACTIVATED CONDITION AND ACQUIRING THE SIGNAL FROM THE CONCENTRATE SENSOR 30 TO DETERMINE THE CONCENTRATION "Ceff" OF THE SUBSTANCE INTO THE EFFLUENT FLUID.

↓

RECEIVING A FLOW RATE "Qdia" OF THE DIALYSIS FLUID, A FLOW RATE "Qeff" OF THE EFFLUENT FLUID AND CALCULATING A SOLUTE REMOVAL RATE "J" OF SAID SUBSTANCE FROM: J = Qeff x Ceff - Qdia x Cdia

↓

DECREASING A RATIO OF THE FLOW RATE "Qeff" OF THE EFFLUENT FLUID TO THE BLOOD FLOW RATE "Qb" FOR A TIME "T" TO GET EQUILIBRIUM BETWEEN THE CONCENTRATION "Ceff_eq" OF THE SUBSTANCE INTO THE EFFLUENT FLUID AND A BLOOD CONCENTRATION "Cb" OF THE SAME SUBSTANCE AT THE INLET 3a OF THE PRIMARY CHAMBER 3.

↓

ASSUMING THE CONCENTRATION "Ceff_eq" OF THE SUBSTANCE INTO THE EFFLUENT FLUID EQUAL TO THE PLASMA WATER CONCENTRATION OF THE SOLUTE "Cpw" OF THE SUBSTANCE AT THE INLET 3A OF THE PRIMARY CHAMBER 3.

↓

CALCULATING THE DIALYSANCE "Dpw" OF THE SUBSTANCE REFERRED TO THE PLASMA WATER CONCENTRATION FROM: Dpw = (Qeff x Ceff - Qdia x Cdia) / Ceff_eq - Cdia)

FIG.6

CLOSING THE VALVE 32 AND OPENING THE ANOTHER FEED VALVE 35.
DRIVING THE PRE-BLOOD PUMP 20 AT ITS NOMINAL RATE AND DRIVING THE FLUID PUMP ASSEMBLY 29 IN THE FIRST ACTIVATED CONDITION TO CAUSE THE REPLACEMENT SOLUTION OF THE FIRST SOURCE 19 TO FLOW TOWARDS THE EFFLUENT LINE 10.
ACQUIRING THE SIGNAL FROM THE CONCENTRATE SENSOR 30 AND DETERMINING THE CONCENTRATION OF THE SUBSTANCE INTO THE REPLACEMENT SOLUTION OF THE FIRST SOURCE 19.

FIG.7

CLOSING THE VALVE 32 AND OPENING THE VALVE 43.
DRIVING THE FLUID PUMP 29 IN THE FIRST ACTIVATED CONDITION TO RINSE THE BY-PASS LINE 28 AND THE CONCENTRATE SENSOR 30.
DISCHARGING THE RINSING FLUID TO THE DRAINAGE 12.

11
27
13
6b
3b
29
57
59
13
9
4a
3
55
4
10
5
58
54
60
56
4b
30
14
3a
6a
28
14
12

FIG.8

FIG.9

27
29
30
14
2
6a
7
10
6b
12
24
28
23
32
18

FIG.10

23
28
32
28
24
18
29
30
2
14
12
7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014190876 A
- US 5399157 A
- US 5744031 A
- US 6623442 B
- US 2014098359 A
- WO 2021205389 A
- US 5685988 A1
- EP 1029554 A2